# FASCICULE DE BREVET EUROPEEN

(11) **EP 1 684 788 B1**
(45) Date de publication et mention de la délivrance du brevet: **03.09.2008**
(21) Numéro de dépôt: 04805493.6
(22) Date de dépôt: 19.11.2004
(51) Int. Cl.: A61K 38/12, C07K 11/02, C07K 7/64, A61P 15/08, C07K 5/12, C12Q 1/00, C07K 14/705, G01N 33/68

(54) **PEPTIDE AUGMENTANT LA CAPACITE FUSIOGENE D'UNE GAMETE**
PEPTID-ERHÖHENDE FUSIOGENE KAPAZITÄT EINES GAMETEN
PEPTIDE INCREASING FUSIOGENIC CAPACITY OF A GAMETE

(30) Priorité: 19.11.2003 FR 0313545
(43) Date de publication de la demande: 02.08.2006
(73) Titulaire: Universite Paris 13, 93430 Villetaneuse (FR); CENTRE NATIONAL DE LA RECHERCHE SCIENTIFIQUE (CNRS), 75794 Paris Cedex 16 (FR)
(72) Inventeur: BOMSEL, Morgane, F-75003 Paris (FR); WOLF, Jean-Philippe, F-75013 Paris (FR)
(74) Mandataire: Gallois, Valérie
(86) Numéro de dépôt international: PCT/FR2004/002956
(87) Numéro de publication internationale: WO 2005/051799

(56) Documents cités:
- WO-A-96/00581
- US-A- 5 849 865
- EVANS JANICE P ET AL: "Mouse sperm-egg plasma membrane interactions: Analysis of roles of egg integrins and the mouse sperm homologue of PH-30 (fertilin) beta" JOURNAL OF CELL SCIENCE, vol. 108, no. 10, 1995, pages 3267-3278, XP002289784 ISSN: 0021-9533 cité dans la demande
- MYLES DIANA G ET AL: "Identification of a binding site in the disintegrin domain of fertilin required for sperm-egg fusion" PROCEEDINGS OF THE NATIONAL ACADEMY OF SCIENCES OF THE UNITED STATES OF AMERICA, vol. 91, no. 10, 1994, pages 4195-4198, XP002289785 ISSN: 0027-8424 cité dans la demande
- GUPTA S ET AL: "Characterization of fertilin beta-disintegrin binding specificity in sperm-egg adhesion." BIOORGANIC & MEDICINAL CHEMISTRY. APR 2000, vol. 8, no. 4, avril 2000 (2000-04), pages 723-729, XP002289786 ISSN: 0968-0896 cité dans la demande
- BRONSON R A ET AL: "Evidence that a functional fertilin-like ADAM plays a role in human sperm-oolemmal interactions." MOLECULAR HUMAN REPRODUCTION. MAY 1999, vol. 5, no. 5, mai 1999 (1999-05), pages 433-440, XP002289787 ISSN: 1360-9947 cité dans la demande

### SEQUENCE LISTING

<110> université Paris 13, Centre National de Recherche scientifique
<120> PEPTIDE AUGMENTANT LA CAPACITE FUSIOGENE D'UNE GAMETE
<130> B0200WO
<150> FR 03 13545
   <151> 2003-11-19
<160> 9
<170> PatentIn version 3.1
<210> 1
   <211> 30
   <212> PRT
   <213> Homo sapiens
<220>
   <221> SITE
   <222> (14)..(16)
   <223> Tripeptide
<400> 1
   Cys Leu Phe Met Ser Lys Glu Arg Met Cys Arg Pro Ser Phe Glu Glu
   1               5                   10                  15
   Cys Asp Leu Pro Glu Tyr Cys Asn Gly Ser Ser Ala Ser Cys
               20                  25                  30
<210> 2
   <211> 30
   <212> PRT
   <213> Mus musculus
<220>
   <221> SITE
   <222> (14)..(16)
   <223> Tripeptide
<400> 2
   Cys Lys Leu Lys Arg Lys Gly Glu Val Cys Arg Leu Ala Gln Asp Glu
   1               5                   10                  15
   Cys Asp Val Thr Glu Tyr Cys Asn Gly Thr Ser Glu Val Cys
               20                  25                  30
<210> 3
   <211> 30
   <212> PRT
   <213> Cavia porcellus
<220>
   <221> SITE
   <222> (14)..(16)
   <223> Tripeptide
<400> 3
   Cys Glu Phe Lys Thr Lys Gly Glu Val Cys Arg Glu Ser Thr Asp Glu
   1               5                   10                  15
   Cys Asp Leu Pro Glu Tyr Cys Asn Gly Ser Ser Gly Ala Cys
               20                  25                  30
<210> 4
   <211> 30
   <212> PRT
   <213> oryctolagus cuniculus
<220>
   <221> SITE
   <222> (14)..(16)
   <223> Tripeptide
<400> 4
   Cys Thr Phe Lys Glu Arg Gly Gln Ser Cys Arg Pro Pro Val Gly Glu
   1               5                   10                  15
   Cys Asp Leu Phe Glu Tyr Cys Asn Gly Thr Ser Ala Leu Cys
               20                  25                  30
<210> 5
   <211> 30
   <212> PRT
   <213> Macaca fascicularis
<220>
   <221> SITE
   <222> (14)..(16)
   <223> Tripeptide
<400> 5
   Cys Leu Phe Met Ser Gln Glu Arg Cys Cys Arg Pro Ser Phe Asp Glu
   1               5                   10                  15
   Cys Asp Leu Pro Glu Tyr Cys Asn Gly Thr Ser Ala Ser Cys
               20                  25                  30
<210> 6
   <211> 30
   <212> PRT
   <213> Bos taurus
<220>
   <221> SITE
   <222> (14)..(16)
   <223> Tripeptide
<400> 6
   Cys Ala Phe Ile Pro Lys Gly His Ile Cys Arg Gly Ser Thr Asp Glu
   1               5                   10                  15
   Cys Asp Leu His Glu Tyr Cys Asn Gly Ser Ser Ala Ala Cys
               20                  25                  30
<210> 7
   <211> 30
   <212> PRT
   <213> Rattus norvegicus
<220>
   <221> SITE
   <222> (14)..(16)
   <223> Tripeptide
<400> 7
   Cys Asn Leu Lys Ala Lys Gly Glu Leu Cys Arg Pro Ala Asn Gln Glu
   1               5                   10                  15
   Cys Asp Val Thr Glu Tyr Cys Asn Gly Thr Ser Glu Val Cys
               20                  25                  30
<210> 8
   <211> 30
   <212> PRT
   <213> Sus scrofa
<220>
   <221> SITE
   <222> (14)..(16)
   <223> Tripeptide
<400> 8
   Cys Ser Phe Met Ala Lys Gly Gln Thr Cys Arg Leu Thr Leu Asp Glu
   1               5                   10                  15
   Cys Asp Leu Leu Glu Tyr Cys Asn Gly Ser Ser Ala Ala Cys
               20                  25                  30
<210> 9
   <211> 6
   <212> PRT
   <213> artificial sequence
<220>
   <223> peptide FEEc
   <220>
<221> DISULFID
   <222> (1)..(6)
   <223>
<400> 9
   Cys Ser Phe Glu Glu Cys
   1               5

## Description

### DOMAINE TECHNIQUE

La présente invention se rapporte à des peptides cycliques augmentant la capacité fusiogène de l'ovocyte et/ou du spermatozoïde, à des compositions pharmaceutiques les comprenant et à leurs utilisations, notamment pour supplémenter des milieux de culture utilisés pour la réalisation de la Fécondation in Vitro. Les peptides de l'invention comprennent typiquement un domaine issu de la boucle disintégrine de la fertiline béta.

### ETAT DE LA TECHNIQUE

La fécondation est un processus complexe aboutissant à la fusion des gamètes (spermatozoïde et ovocyte) pour former un embryon. Ce processus naturel a lieu dans l'ampoule tubaire et aboutit à une grossesse après migration de l'embryon dans la cavité utérine.

Lorsque le couple est stérile, des techniques d'Assistance Médicale à la Procréation sont mises en jeu pour obtenir *in vitro* des embryons. Ils sont alors transférés par voie basse dans l'utérus où ils s'implantent.

La fécondation *in vitro* consiste simplement à mettre en présence, dans un milieu de culture adapté et dans des conditions de pH et de température appropriées, ovocytes et spermatozoïdes pour que la fécondation puisse se produire. Les techniques ont été largement développées depuis 25 ans (Bavister 2002).

Quand, pour des raisons parfois non élucidées, des échecs de fécondation ont lieu, les biologistes ont recours aux techniques de fécondation assistée qui consistent à microinjecter un spermatozoïde directement dans le cytoplasme de l'ovocyte. L'évolution des embryons microinjectés n'est toutefois pas aussi bonne que celle observée après fécondation spontanée. En effet, les taux de grossesse obtenus par transfert de ces embryons sont inférieurs à ceux obtenus après fécondation in vitro simple (25,4 % vs 26,5 %, sur plus de 20 000 tentatives) (FIVNAT, 2001). De même, les embryons issus de microinjection supportent moins bien la congélation que ceux issus de FIV (BLEFCO 2001, Simon et al., 1998). Ainsi, une lyse embryonnaire plus importante se produit au moment de la décongélation et un taux d'implantation plus faible est obtenu.

Enfin nous savons que l'activation de l'ovocyte consécutive à la fécondation est médiée par des ondes calciques induites par un facteur spermatique (Swan 1999). Ces ondes ont une amplitude et une fréquence réglées dont dépend la qualité embryonnaire (Swan1999). Le court-circuit des étapes membranaires de l'interaction gamétique modifie le régime de ces ondes calciques pour deux raisons. L'interaction physiologique entre les membranes n'a pas lieu, et le traumatisme cellulaire dû au passage de la pipette de microinjection dans le cytoplasme provoque la sortie de calcium des pools de réserve (Tésarik et al ; 2002.). Cette sortie, si elle assure l'activation de l'ovocyte, peut aussi perturber le développement ultérieur de l'embryon.

L'étude du processus de fusion des membranes spermatique et ovocytaire au moment de la fécondation a permis de mettre en évidence en partie le mécanisme membranaire de la fusion.

La formation d'un complexe moléculaire membranaire a lieu à la surface de l'ovocyte. Ce complexe est induit par le spermatozoïde au moment de la fécondation. Sa composition et son mécanisme d'action sont partiellement connus, mais l'on sait que l'inhibition de leur formation entraîne l'inhibition de la fusion gamétique. La fécondation est donc bien liée à la formation de ces patches à la surface de l'ovocyte.

L'hypothèse communément admise est que le spermatozoïde interagit par le biais d'un premier récepteur avec la membrane et qu'après transduction d'un signal transmembranaire un mécanisme cellulaire permet la constitution de ces patches sur lesquels le spermatozoïde vient se fixer.

Des études ont lieu pour déterminer la nature du ligand spermatique et du récepteur ovocytaire impliqués. Il existe une protéine de membrane spermatique du nom de Fertiline (Evans 2002). Cette molécule est un dimère alpha béta dont les molécules appartiennent à la famille des protéines ADAM (A Disintegrine And Metalloprotease) (Evans 2001). La présence d'intégrines a été mise en évidence sur l'ovocyte. Par l'intermédiaire de son site de liaison putatif, la fertiline spermatique est susceptible d'interagir avec une ou des intégrines ovocytaires.

Les molécules de membranes appelées intégrines sont des molécules de liaison qui interviennent dans les liaisons cellule-matrice extra-cellulaire et cellule-cellule. Leurs ligands se lient à leur domaine extracellulaire par l'intermédiaire d'un site le liaison constitué par un tripeptide situé au sommet d'une boucle.

Les spermatozoïdes possèdent une molécule de membrane ayant un site disintégrine capable de se lier à une intégrine. Cette molécule, découverte chez le cobaye, mais présente chez tous les mammifères étudiés dont l'homme et la souris s'appelle Fertiline. La Fertiline humaine a été séquençée, ce qui fait que son site putatif de liaison est connu. Il s'agit du tripeptide FEE (Phénylalanine, Acide glutamique, Acide glutamique) (Gupta et al. 1995, Vidaeus et al., 1997). Un octapeptide linéaire contenant la séquence FEE inhibe l'adhérence des spermatozoïdes à l'ovocyte et leur pénétration (Bronson 1999). Des effets inhibiteurs comparables ont été observés dans d'autres espèces avec des peptides linéaires et cycliques (Mwethera 1999 ; Gupta 2000 ; Li 2002 ; Myles 1994). Evans (1995) rapporte une absence d'effet inhibiteur des peptides cycliques chez la souris.

### EXPOSE DE L'INVENTION

Au cours de recherches d'inhibteurs de la fécondation, les inventeurs ont synthétisé un peptide cyclique, appelé FEEc, reproduisant le site de liaison de la fertiline humaine béta et ont testé son action sur les ovocytes humains. Contrairement à leur attente et de manière surprenante, les inventeurs ont ainsi mis en évidence que (1) le peptide cyclique FEEc se fixe sur la membrane de l'ovocyte humain, (2) qu'il induit une relocalisation des protéines d'adhésion à la surface de l'ovocyte, cette relocalisation étant normalement induite par les spermatozoïdes, (3) qu'il augmente la capacité fusiogène des ovocytes. Ces résultats ont été corroborés par des expériences chez la souris.

Ainsi, les inventeurs ont identifié une nouvelle classe de peptides cycliques capables d'augmenter la capacité fusiogène des ovocytes et/ou des spermatozoïdes. Cette classe de peptides cycliques serait en outre capable d'activer l'ovocyte.

L'invention concerne l'utilisation d'un peptide cyclique comprenant le site de liaison de la fertiline béta (ADAM 2) à l'intégrine de l'ovocyte pour augmenter la capacité fusiogène et/ou pour activer d'un ovocyte, typiquement in vitro ou ex vivo. Ce site de liaison est contenu dans la boucle disintégrine de la fertiline béta. Ce peptide cyclique comprend au minimum le tripeptide indispensable à cette liaison. Ce tripeptide varie suivant les espèces. Cependant, l'organisation de la boucle disintégrine est très bien conservée parmi les espèces (voir Table 1). Il sera donc facile à l'homme du métier de définir la boucle disintégrine de la fertiline béta et d'identifier le tripeptide.

**Table 1 : Boucle disintégrine**

| | | |
|---|---|---|
| Homme | ₍₄₃₆₎**C**LFMSKERM**C R**PS**FEE**CDLP **EYCNG**S**S**AS**C**₍₄₆₅₎ | SEQ ID No 1 |
| Souris | ₍₄₄₀₎**C**KLKRKGEV**C R**LA**QDECD**VT **EYCNG**T**S**EV**C**₍₄₆₉₎ | SEQ ID No 2 |
| Cobaye | ₍₄₃₃₎**C**EFKTKGEV**C R**ES**TDECD**LP **EYCNG**S**S**GA**C**₍₄₆₂₎ | SEQ ID No 3 |
| Lapin | ₍₄₃₅₎**C**TFKERGQS**C R**PP**VGECD**LF **EYCNG**T**S**AL**C**₍₄₆₄₎ | SEQ ID No 4 |
| Macaque | ₍₄₃₆₎**C**LFMSQERC**C R**PS**FDECD**LP **EYCNG**T**S**AS**C**₍₄₆₅₎ | SEQ ID No 5 |
| Bovin | ₍₄₃₅₎**C**AFIPKGHI**C R**GS**TDECD**LH **EYCNG**S**S**AA**C**₍₄₆₄₎ | SEQ ID No 6 |
| Rat | ₍₄₄₁₎ **C**NLKAKGEL**C R**PA**NQECD**VT **EYCNG**T**S**EV**C**₍₄₇₀₎ | SEQ ID No 7 |
| Porc | ₍₄₃₅₎**C**SFMAKGQT**C R**LT**LDECD**LL **EYCNG**S**S**AA**C**₍₄₆₄₎ | SEQ ID No 8 |

| | | |
|---|---|---|
| *Les positions indiquées entre parenthèse correspondent aux positions dans les séquences fertilines béta dont les références sont données plus loin. Les résidus en gras soulignés correspondent au tripeptide. Les résidus en gras sont parfaitement conservés parmi les espèces.* | | |

Une séquence consensus de la boucle intégrine peut être déduite de la Table 1.
C-X₂-(F/L)-(K/M/I)-X₅-(KJR/Q)-(G/E)-X₈-X₉-C-R-X₁₂-X₁₃-**TriPept**-C-D-(L/V)-X₂₀-E-Y-C-N-(G/E)-(T/S)-S-(A/E/G)-X₂₉-C
où les groupes X représentent, indépendamment les uns des autres, un acide aminé et « TriPept » le tripeptide indispensable à la liaison de la fertiline à l'intégrine. De préférence, X₈ est un acide aminé chargé. Plus particulièrement, il est sélectionné parmi E, R, et Q. De préférence, X₁₂ est sélectionné parmi P, L, E, et G. De préférence, X₁₃ est un acide aminé peu volumineux et non chargé, plus particulièrement sélectionné parmi S, A, P, et T. De préférence, X₂₉ est un acide aminé peu volumineux et non chargé, plus particulièrement sélectionné parmi S, A, et V.

Le peptide cyclique peut être cyclisé par tout moyen connu par l'homme du métier. Le peptide peut être cyclisé par l'intermédiaire d'une liaison covalente entre la chaine principale et la chaîne principale, entre la chaine principale et une chaine latérale, ou entre une chaîne latérale et une autre chaîne latérale. Cette liaison peut être une liaison disulfure, amide ou thioéther.

Par exemple, le peptide peut être cyclisé par une liaison peptidique entre le résidu N-terminal et le résidu C-terminal, ou avec des groupements amines ou carboxyliques des chaines latérales de résidus.

De préférence, le peptide est cyclisé par l'intermédiaire de deux résidus cystéine, plus particulièrement par l'intermédiaire d'un pont disulfure entre ces deux résidus cystéines. La localisation des résidus cystéine doit permettre la cyclisation du peptide. Les résidus cystéine peuvent être placés de telle sorte qu'après cyclisation, le peptide présente une queue polypeptidique. De préférence, ces résidus cystéine seront placés aux extrémités du peptide.

Le peptide cyclique selon la présente invention peut donc être décrit par la formule suivante : 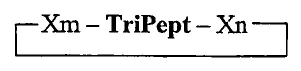 où X représente un acide aminé, m et n est compris entre 0 et 14. Comme indiqué précédemment, dans les formules de l'invention, les groupes X sont indépendants les uns des autres et peuvent représenter, au sein d'une même molécule, des acides aminés différents ou identiques. De préférence, lorsque m ou n est égal à 0, l'autre est au moins 1. De préférence, m+n est inférieur à 10, de préférence inférieur ou égal à 5. Dans un mode de réalisation préféré, m+n est égal à 3. De préférence, le tripeptide présente une séquence X-(Q/D/E)-E, de préférence X-(D/E)-E. Par exemple, le tripeptide peut être sélectionné par le groupe constitué de (Q-D-E), (F-E-E), (T-D-E), (V-G-E), (F-D-E), (T-D-E), (N-Q-E), (L-D-E). Dans un mode de réalisation préféré, le tripeptide est (F-E-E).

De préférence, le peptide cyclique selon la présente invention est décrit par la formule suivante :

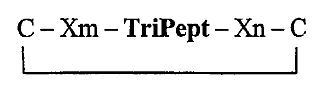

Les résidus cystéine impliqués dans la cyclisation du peptide peuvent se trouver naturellement dans la boucle disintégrine ou peuvent être introduits dans la séquence du peptide. Les boucles disintégrines sont riches en cystéines. En effet, des résidus cystéine sont conservés en positions 1, 10, 17, 23 et 30 de ces boucles. Ainsi, les peptides peuvent être cyclisés par l'intermédiaire d'un pont disulfure choisi parmi le groupe suivant : C1-C17, C1-C23, C1-C30, C10-C17, C10-C23, et C10-C30. De préférence, les peptides sont cyclisés par l'intermédiaire d'un pont disulfure choisi parmi C10-C17 et C10-C23.

Les peptides cycliques selon la présente invention peuvent donc présenter une des structures suivantes :
C₁-X2-(F/L)-(K/M/I)-X₅-(K/R/Q)-(G/E)-X₈-X₉-C-R-X₁₂-X₁₃-**TriPept**-C₁₇;
C₁-X2-(F/L)-(K/M/I)-X₅-(K/R/Q)-(G/E)-X₈-X₉-C-R-X₁₂-X₁₃-**TriPept**-C-D-(L/V)-X₂₀-E-Y-C₂₃;
C₁-X2-(F/L)-(K/M/I)-X₅-(K/R/Q)-(G/E)-X₈-X₉-C-R-X₁₂-X₁₃-**TriPept**-C-D-(L/V)-X₂₀-E-Y-C-N-(G/E)-(T/S)-S-(A/E/G)-X₂₉-C₃₀;
C₁₀- R-X₁₂-X₁₃-**TriPept**-C₁₇;
C₁₀- R-X₁₂-X₁₃-**TriPept**-C-D-(L/V)-X₂₀-E-Y-C₂₃;
C₁₀- R-X₁₂-X₁₃-**TriPept**-C-D-(L/V)-X₂₀-E-Y-C-N-(G/E)-(T/S)-S-(A/E/G)-X₂₉-C₃₀;
où X représentant un acide aminé et les résidus cystéines des extrémités des peptides formant des ponts disulfures.

De préférence, les peptides cycliques selon la présente invention présentent une des structures suivantes :
C₁₀- R-X₁₂-X₁₃-**TriPept**-C₁₇; ou
C₁₀- R-X₁₂-X₁₃-**TriPept**-C-D-(L/V)-X₂₀-E-Y-C₂₃.

Plus particulièrement, les peptides cycliques selon l'invention sont choisis parmi un groupe constitué des fragments 1-17, 1-23, 1-30, 10-17, 10-23, et 10-30 d'une des séquences SEQ ID Nos 1-8, de préférence de la séquence SEQ ID No 1. De préférence, les peptides cycliques selon l'invention sont choisis parmi un groupe constitué des fragments 10-17, 10-23 d'une des séquences SEQ ID Nos 1-8, de préférence de la séquence SEQ ID No 1.

Les résidus cystéine peuvent être introduits dans le peptide à cycliser. Dans un mode de réalisation préféré, le peptide selon l'invention a la structure suivante : 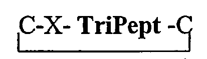 où X est un acide aminé. Un acide aminé peu encombrant et non chargé sera privilégié. De préférence, X est choisi parmi A, S ou T. Plus particulièrement, X est A ou S. Dans un mode de réalisation préféré, le X est la sérine et le tripeptide présente la séquence (F-E-E) (SEQ ID No 9). L'invention concerne tout particulièrement ce peptide cyclique (dénommé FEEc dans les exemples) et son utilisation pour augmenter la capacité fusiogène et/ou activer les ovocytes.

Les acides aminés du peptide cyclique selon la présente invention peuvent être naturels ou non. Par acide aminé non-naturel est entendu un analogue ou dérivé d'un acide aminé naturel. Par exemple, un acide aminé non naturel peut présenter une chaine latérale allongée, plus courte, ou variante présentant des groupements fonctionnels appropriés. Bien entendu, les stéréoisomères L et D sont envisagés. De plus, les liaisons peptidiques peuvent être modifiées pour les rendre résistantes à la protéolyse. Par exemple, au moins une liaison peptidique (-CO-NH-) peut être remplacée par une liaison divalente choisie parmi (-CH₂-NH-), (-NH-CO-), (-CH₂-O-), (-CH₂-S-), (-CH₂-CH₂-), (-CO-CH₂-), (-CHOH-CH₂-), (-N=N-), et (-CH=CH-).

Les résidus des séquences décrites ci-dessus peuvent varier de façon conservative. Par conservative est entendu que le résidu variant présentera des caractéristiques physicochimiques comparables. Parmi les caractéristiques physicochimiques prises en compte, on trouve l'encombrement stérique, la polarité l'hydrophobie, ou la charge.

Par conséquent, la présente invention concerne également les variants et/ou les dérivés de ces peptides cycliques et leur utilisation, notamment pour augmenter la capacité fusiogène et/ou activer les ovocytes. Ces variants et dérivés concervent la capacité de liaison à l'intégrine de l'ovocyte, plus particulièrement à l'intégrine α6β1.

L'invention concerne également un multimère de peptide cyclique selon l'invention. Cette polymérisation du peptide cyclique peut être réalisée par tout moyen disponible à l'homme du métier. De préférence, le peptide cyclique est couplé à une molécule porteuse permettant la polymérisation du peptide. La liaison entre le peptide cyclique et la molécule porteuse peut être covalente ou non-covalente. Les méthodes pour fixer les peptides cycliques à la molécule porteuse sont bien connues de l'homme du métier et comprennent la chimie des amines, le couplage carbodiimide des dérivées carboxyl et amino, l'activation du bromure de cyanogène, de N-hydroxysuccinimide, d'époxide, de sulfhydryl, ou d'hydrazide. La liaison entre la molécule porteuse et les peptides cycliques peut être directe ou indirecte. Lorsqu'elle est indirecte, elle peut se faire à travers un lien. Ce lien peut avoir un rôle d'espaceur qui évite une interaction de la molécule porteuse sur les propriétés du peptide cyclique, notamment les propriétés fusiogènes et/ou activatrices. Ce lien peut être un peptide. Le peptide cyclique doit être fixé à la molécule porteuse de manière à maintenir l'accessibilité du tripeptide. Le nombre de peptides cycliques compris dans le multimère est de préférence compris entre 2 et 1000. La forme multimérique du peptide selon la présente invention permet d'augmenter l'effet du peptide cyclique sur la relocalisation des protéines d'adhésion de l'ovocyte.

Par exemple, la polymérisation peut être faite par l'intermédiaire du couple biotine/streptavidine qui permet de préparer un tétramère du peptide cyclique. Ainsi, chaque peptide cyclique est lié à une biotine et quatre biotines peuvent se lier à une molécule de streptavidine.

Alternativement, plusieurs peptides cycliques selon la présente invention peuvent être portés par une protéine transporteuse. De préférence, cette protéine n'a pas d'effet biologique sur l'ovocyte. L'homme du métier connait plusieurs protéines utilisées comme protéine porteuse dont l'albumine sérique bovine (SAB) et l'hémocyanine de patelle.

D'autre part, plusieurs peptides cycliques selon la présente invention peuvent être immobilisés sur un support solide. Le support solide peut être de façon non exhaustive de l'agarose, du verre, des résines cellulosiques, des résines de silice, du polystyrène, du polyacrylamide. Le support solide peut être modifié avec des groupements fonctionnels permettant la fixation des peptides cycliques, par exemple par l'intermédiaire des groupements carboxyl, amino, sulfhydryl, hydroxyl et/ou carbohydrate contenus dans ces peptides.

La présente invention concerne une composition comprenant un peptide cyclique selon la présente invention ou un multimère de celui-ci. Cette composition peut être un milieu destiné à la culture de gamètes. Cette composition peut comprendre en outre un support pharmaceutiquement acceptable. Cette composition peut être une composition pharmaceutique, de préférence adaptée à une application locale. De préférence, cette application locale est faite au niveau du tractus génital femelle.

La présente invention concerne l'utilisation in vitro ou ex vivo d'un peptide cyclique selon la présente invention, d'un multimère de celui-ci ou d'une composition selon la présente invention pour augmenter la capacité fusiogène d'une gamète, en particulier de l'ovocyte, ou pour activer un ovocyte, ainsi qu'une méthode correspondante.

Notamment, les peptides cycliques selon la présente invention peuvent être utilisés pour la fécondation in vitro, pour l'insémination artificielle, et pour le transfert nucléaire (clonage) chez les mammifères, de préférence non-humains. De préférence, ces peptides seront utilisés pour réaliser ou améliorer une fécondation in vitro, plus particulièrement chez les humains. A cet égard, l'invention concerne également un milieu de culture de gamètes supplémenté par un peptide cyclique selon la présente invention ou un multimère de celui-ci. Dans un mode de réalisation particulier, l'ovocyte est mis en présence du peptide cyclique selon l'invention avant d'être incubé en présence des spermatozoïdes. Dans un autre mode de réalisation, l'ovocyte est mis en présence simultanément avec le peptide cyclique selon la présente invention et le gamète male.

Les peptides de l'invention peuvent être également utilisés dans un traitement destiné à augmenter la fécondité. Ainsi la présente invention concerne l'utilisation d'un peptide selon la présente invention pour la préparation d'un médicament destiné à traiter les problèmes de fécondité, ainsi qu'une méthode correspondante.

Les peptides cycliques selon la présente invention peuvent également être utilisés pour potentialiser les spermatozoïdes.

Les différentes méthodes et utilisations de l'invention peuvent être mises en oeuvre in vitro ou ex vivo . Elles sont avantageusement pratiquées in vitro ou ex vivo afin de produire des cellules ou matériels biologiques traités.

Les peptides cycliques selon la présente invention peuvent être utilisés pour toute espèce dont la reproduction fait appel à des gamètes. L'invention présente un intérêt tout particulier pour les espèces en voie de disparition, les espèces peu fécondes ou les espèces de grande valeur. Plus particulièrement, l'invention considère l'utilisation des peptides cycliques chez les mammifères. De préférence, la présente invention peut s'appliquer à la reproduction ou au clonage des ovins, des bovins, des équins, etc... Bien entendu, la présente invention s'applique également dans l'aide à la procréation humaine.

Ce peptide ajouté dans les milieux de culture des gamètes humains est susceptible de potentialiser leur capacité fusiogène et d'amener à la constitution d'embryons tout en respectant les interactions physiologiques membranaires. Il y a deux intérêts potentiels à son utilisation : la réduction de l'utilisation de la microinjection intracytoplasmique comme technique de fécondation et l'amélioration de la qualité des embryons obtenus.

D'autres aspects et avantages de l'invention apparaîtront à la lecture des exemples qui suivent, qui doivent être considérés comme illustratifs et non limitatifs.

### EXEMPLES

### Résultats de l'étude du FEE cyclique sur la fusion gamétique chez l'homme

### Matériel et méthodes

### Les ovocytes humains

Les ovocytes humains utilisés pour les expérimentations avaient deux origines : les ovocytes en échec de fécondation après FIV et les ovocytes non microinjectés car immatures au moment de l'ICSI et maturés in vitro. Les patients avaient donné ces ovocytes à la recherche et avaient signé à cette fin un consentement visé par le CCPPRB de l'hôpital d'Aulnay sous Bois.

Ces ovocytes âgés de 48 heures ou maturés *in vitro* ne sont pas ou plus capables de donner un embryon. Par ailleurs le mécanisme du blocage à la polyspermie qui assure la qualité diploïde de l'embryon est situé au niveau de la zone pellucide de l'ovocyte. En dépellucidant ces ovocytes, on enlève ce mécanisme protecteur ce qui aboutit en cas de fusion gamétique à des zygotes polyspermiques non viables. Enfin, le développement embryonnaire se faisant à l'intérieur de la zone pellucide pendant les 6 premiers jours, son absence empêche un tel développement d'avoir lieu.

Pour garder la membrane ovocytaire aussi proche que possible de son état naturel, la dépellucidation que les inventeurs ont faite a été réalisée de façon mécanique à l'aide de ciseaux de microchirurgie.

### Les spermatozoïdes

Le sperme de donneurs féconds a été collecté après 3 jours d'abstinence et préparé comme pour les FIV. Brièvement, l'éjaculat a été gardé à 37° jusqu'à liquéfaction puis sélectionné sur un gradient de Puresperm 2 couches (90 et 45 %). Le sperme a été alors gardé dans des conditions capacitantes jusqu'à l'insémination des ovocytes.

### Le peptide synthétique cyclique FEEc

La séquence complète du domaine disintégrine de la Fertiline humaine constituant la boucle est représentée ci-dessous :

| | |
|---|---|
| CLFMSKERMCRP**SFEECD**LPEYCNGSSASC | SEQ ID No 1 |

Le peptide synthétique cyclique FEEc utilisé dans cet exemple est le suivant :

| | |
|---|---|
| CS**FEEC** | SEQ ID No 9 |

Le peptide FEEc que les inventeurs ont synthétisé comprend la séquence du tripeptide FEE. Il comprend la Sérine qui le précède et la Cystéine qui le suit. Les inventeurs ont ajouté une Cystéine supplémentaire en amont du site de liaison. Les deux cystéines situées aux extrémités du peptide permettent de cycliser le peptide.

### Immunofluorescence

La sous-unité intégrine alpha 6 est présente sur la membrane de l'ovocyte humain. Elle fait partie du complexe multimoléculaire de fusion. C'est la raison pour laquelle les inventeurs l'ont prise comme témoin du remaniement membranaire.

Les ovocytes dépellucidés ont été incubés dans des gouttes de 20 µl de milieu de culture Ferticult sous huile à température de la pièce. Le milieu a été supplémenté avec un anticorps de souris anti alpha 6 humain à 20 µM (Chemicon International, Londres, GB). Les ovocytes ont alors été lavés puis fixés dans de la PFA 2% pendant une heure. Ils ont ensuite été incubés pendant 45 minutes avec un second anticorps anti-immunoglobuline de souris marqué au FITC ou à la Rhodamine élevé chez l'âne (FITC-conjugated donkey anti-mouse IgG ou Rhodamine-conjugated donkey anti-mouse IgG 10µg/ml ; Jackson laboratories). Après lavage, les ovocytes ont enfin été montés dans de l'Immumount antifade (Shandon Laboratories) entre lame et lamelle et examinés soit au microscope à fluorescence (Zeiss Axiophot) soit au microscope confocal (Leica Lasertechnik, GmbH).

### Tests fonctionnels de fusion

Les test fonctionnels d'inhibition de la fusion ont été réalisés dans des conditions similaires à celles employées pour la FIV : c'est-à-dire dans des conditions suffisamment proches des conditions physiologiques puisqu'elles permettent l'obtention de grossesses et d'enfants. Brièvement, les ovocytes ont été incubés dans des gouttes de 20 µl de Ferticult sous huile pendant 18 heures dans un incubateur sous 5 % de CO2 à 37° avec 4000 spermatozoïdes mobiles. Les tests ont été menés en supplémentant le milieu avec le Peptide FEEc à la concentration de 200 µM. A la fin de l'incubation, les ovocytes ont été lavés et incubés 20 minutes dans du Hoechst 33342 (Sigma) à 5 µg/ml. Après fixation dans du PFA 4% PBS-BSA 1% pendant 30 minutes à température ambiante, les ovocytes ont été montés dans de l'immumount entre lame et lamelle avant d'être examinés sous UV. Les spermatozoïdes fusionnés étaient fluorescents. Les lames ont été analysées sur un microscope Zeiss Axiophot équipé d'une caméra connectée à un logiciel d'analyse d'images Imaging System Package (Applied Imaging, Newcastle-upon-Tyne, UK).

### Résultats

### Immunofluorescence et microscopie confocale.

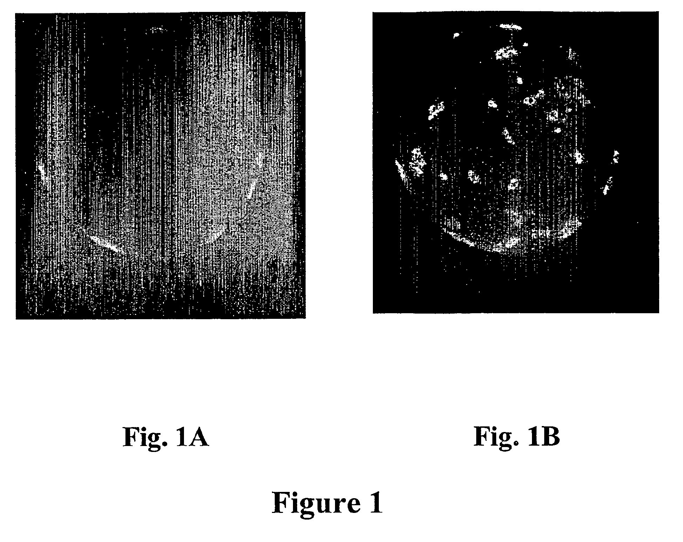

Les ovocytes ont été incubés avec du Peptide FEEc biotinylé 200 µM pendant 45 minutes. L'examen en microscopie confocale a permis de mettre en évidence un marquage membranaire avec le Peptide FEEc comme cela est visible sur la coupe équatoriale de l'ovocyte (Figure 1A). La superposition informatique a montré en outre que ce marquage correspond à des patches membranaires (Fig. 1B).

### Induction de patches de fusion par le Peptide FEEc sur l'ovocyte humain

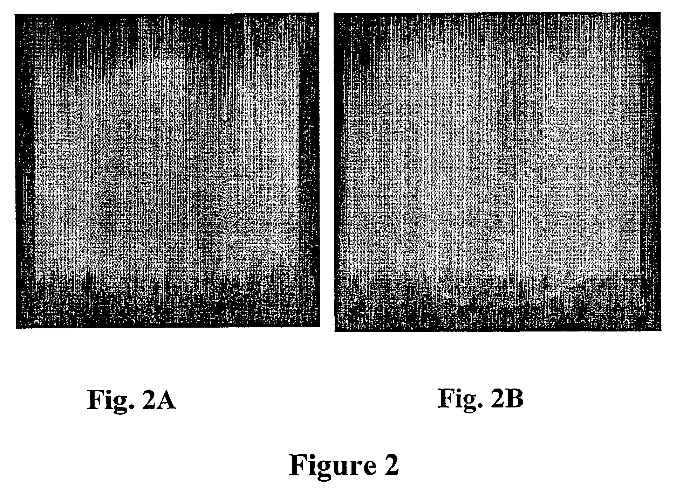

Le spermatozoïde induit des patches multimoléculaires. En incubant des ovocytes maturés in vitro avec une suspension de Peptide FEEc 200 µM pendant 18 heures, les inventeurs ont mis en évidence une redistribution de la sous-unité intégrine α6. En effet, alors que sur l'ovocyte intact la distribution de la sous-unité α6 est homogène sur la surface de l'ovocyte (Fig. 2A), le Peptide FEEc induit sa redistribution sous forme de petites agrégats membranaires. Chez la souris, le spermatozoïde induit effectivement de tels patches lors de la fécondation. Il peut en être conclu que le peptide FEEc induit une redistribution membranaire de protéines d'adhésion comparable à celle qui est induite par le spermatozoïde lui-même au cours de la fécondation.

### Test fonctionnel de fusion avec des gamètes humains

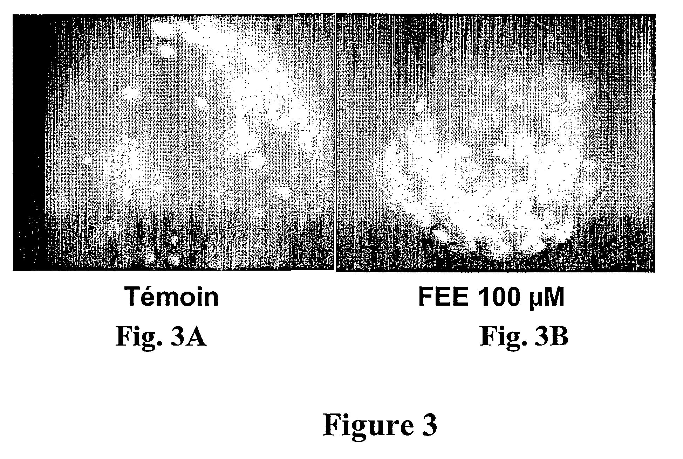

Dans des conditions semblables, des ovocytes dépellucidés ont été incubés en présence de 200 µM de Peptide FEEc et de spermatozoïdes. Dans les ovocytes témoins, une vingtaine de spermatozoïdes fusionnés au sein du cytoplasme ovocytaire ont été observés (Fig. 3A). En présence de 200 µM de Peptide FEEc, le nombre des spermatozoïdes cytoplasmiques a été bien plus important (Fig 3B). L'expérience reproduite sur un plus grand nombre d'ovocytes a fait apparaître une augmentation moyenne de 60% environ du nombre de spermatozoïdes qui ont fusionné avec l'ovocyte (26,1±8,3 vs 16,4±5,2 ; P<0,001). L'augmentation du nombre de spermatozoïdes est donc statistiquement significative. Cet effet est spécifique car l'incubation avec le même peptide brouillé est sans effet. L'action du Peptide FEEc est réversible car les ovocytes préincubés avec le Peptide FEEc puis lavés et inséminés ne montrent pas de variation de leur capacité fusiogène. Il n'y a donc pas non plus d'effet toxique sur les ovocytes mais la co-incubation est nécessaire pour que l'effet du Peptide apparaisse.

### Discussion

Le Peptide FEEc présente donc la propriété d'augmenter la capacité fusiogène de l'ovocyte humain. Il reproduit le mécanisme par lequel le spermatozoïde présent au contact de la membrane ovocytaire induit les complexes moléculaires de fusion ovocytaires et permet donc un processus de fusion plus facile.

Il peut servir à supplémenter les milieux de culture utilisés pour la réalisation de la Fécondation in Vitro. Il devrait alors pouvoir améliorer les taux de fécondation des FIV réalisées pour stérilité idiopathique ou pour déficience spermatique. Il y aurait alors une diminution du recours aux techniques de microinjection et amélioration de la qualité des embryons obtenus.

### Résultats de l'étude du QDE cyclique sur la fusion gamétique chez la souris

De façon semblable à ce que nous avions vu dans l'espèce humaine, nous avons étudié l'effet d'un tripeptide cyclique dans le modèle souris. Par analogie, le motif du tripeptide reproduisait cette fois le site de fixation de la disintégrine de la fertiline β de la souris, c'est à dire le tripeptide QDE. Ce peptide a été appelé QDEc.

### Matériel et méthode.

Des souris C56b1/CBA de 6 semaines ont été superovulées par 5 UI de PMSG et 5 UI d'hCG administrées à 48 heures d'intervalle. Treize heures après la dernière injection les souris ont été sacrifiées et les oviductes ont été récupérés puis dilacérés dans du milieu de M2. Les ovocytes récupérés ont alors été soit inséminés dans leur cumulus soit décoronisés par un bref passage dans de la hyaluronidase. Les ovocytes décoronisés ont ensuite été dépellucidés mécaniquement à l'aide de ciseaux de microdissection sous une loupe binoculaire.

Les ovocytes dépellucidés ont été préincubés 30 min. puis inséminés en présence ; 1) groupe témoin de milieu M16; 2) de milieu supplémenté de 10 µM de QDEc ; 3) 100 µM de QDEc et 4) 1 mM de QDEc. Les ovocytes ont été dans tous les cas inséminés pendant 3 heures avec une concentration de 10⁶ spermatozoïdes mobiles par ml.

Les ovocytes intacts ont été incubés avec QDE cyclique 100 µM et 10⁵ spermatozoïdes/ml.

Après l'incubation, les ovocytes ont été soigneusement lavés puis incubés dans du Hoescht 10 µM pendant 30 min, rincés puis examinés en UV sur un microscope Zeiss. Les données ont été analysées à l'aide du logiciel Statview ^{®}.

### Résultats

### 1- ovocyte dépellucidés

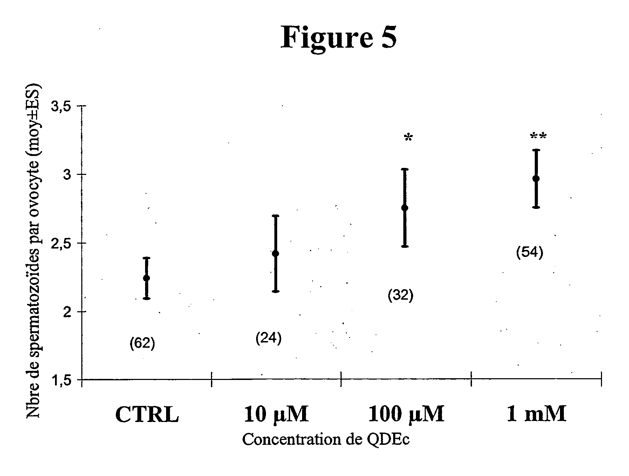

Au cours de 5 expériences, 170 oocytes ont été étudiés en 4 groupes. Alors que la moyenne du nombre de spermatozoïdes fusionnés par ovocyte est de 2,2±0,1 (moy±ES) dans le groupe contrôle, il augmente de façon dose dépendante avec la présence du QDE dans le milieu d'incubation (Figure 5). Il atteint des différences statistiques pour des concentrations de 100 µM (P<0,05) et de façon significative pour 1 mM (P<0,004) avec une augmentation de 32% du nombre des spermatozoïdes fusionnés.

### 2 - ovocytes intacts

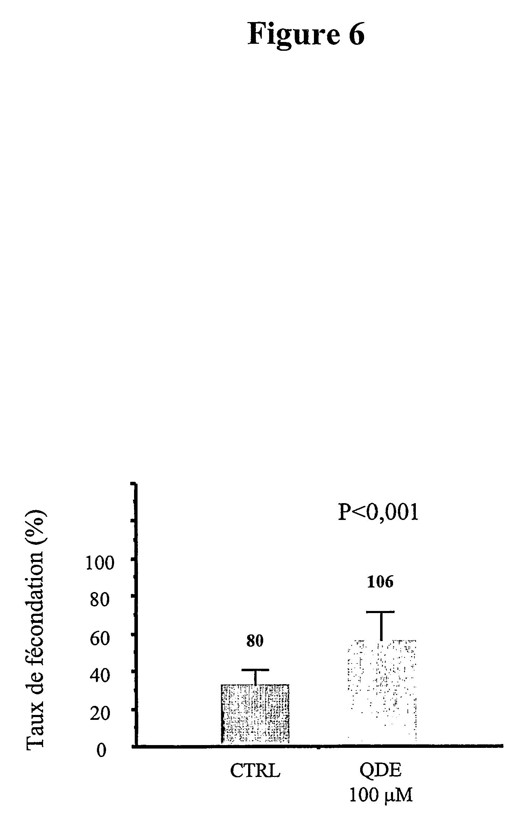

En FIV classique : Les trois expérimentations ont donné des résultats cohérents avec une moyenne de fécondation de 32,1+7,6 % pour le témoin et 56,3+16,0 % pour le groupe testé (P<0,001) (Figure 6).

### Discussion

Le nombre de spermatozoïdes qui fusionnent avec un ovocyte dépellucidé est d'une vingtaine dans l'espèce humaine alors qu'il ne dépasse en règle pas 2 spermatozoïdes chez la souris. L'augmentation de ce nombre, moins importante que dans l'espèce humaine, sous l'effet du QDE atteint un seuil de signification statistique rapidement. De plus, en fécondation in vitro d'ovocytes intacts, le QDE cyclique entraîne une augmentation de 75 % du taux des ovocytes normalement fécondés. Le schéma d'interaction du spermatozoïde avec la membrane ovocytaire semble donc être comparable chez la souris à ce qui se passe dans l'espèce humaine. La version cyclisé du peptide reproduisant le site de fixation du domaine disintégrine de la fertiline β augmente bien les capacités fusiogènes des gamètes.

### Légende des Figures

### Figure 1. Détection du Peptide FEEc biotinylé à la surface de l'ovocyte humain.

Des ovocytes ont été dépellucidés mécaniquement puis incubés pendant 45 min avec le Peptide biotinylé à 100 µM. Le peptide FEEc a été détecté par un anticorps de souris anti-biotine reconnu par un anticorps biotinylé anti-immunoglobuline de souris puis par la streptavidine-FITC. Les ovocytes ont alors été montés dans de l'immumount entre lame et lamelles et examinés au microscope confocal. A : section équatoriale de l'ovocyte, B : superposition des coupes correspondant à un hémi ovocyte.

### Figure 2. Induction de patches de fusion par le Peptide FEEc sur l'ovocyte humain.

Des ovocytes intacts ont été incubés avec du Peptide FEEc 200 µM pendant 45 minutes. Après lavage, ils ont été incubés avec un anticorps anti sous unité intégrine α6 20 µM pendant 45 minutes puis fixés (PFA 4 % pendant 30 minutes). Après lavage, ils ont été incubés avec un second anticorps anti-immunoglobuline de souris marqué à la rhodamine. Ils ont ensuite été examinés en immunofluorescence. Fig.2A : ovocyte témoin montrant une fluorescence diffuse et homogène sur la surface de l'ovocyte. Fig.2B : après incubation avec le Peptide FEEc, il y a une redistribution de la sous-unité α6 sous forme d'agrégats membranaires.

### Figure 3. Test fonctionnel de fusion avec des gamètes humains.

Les ovocytes dépellucidés ont été incubés dans des gouttes de 20 µl sous huile dans du milieu de culture avec 4000 spermatozoïdes mobiles pendant 18 heures. Ils ont ensuite été lavés puis incubés dans une solution de Hoechst 33342 10 µM pendant 30 minutes avant d'être relavés, montés dans de l'immunount entre lame et lamelle et examinés sous UV. Fig.3A : ovocyte témoin montrant la présence d'une vingtaine de spermatozoïdes fusionnés dans le cytoplasme ovocytaire. Fig.3B : ovocyte co-incubés avec le Peptide à 100 µM présentant une soixantaine de spermatozoïdes fusionnés.

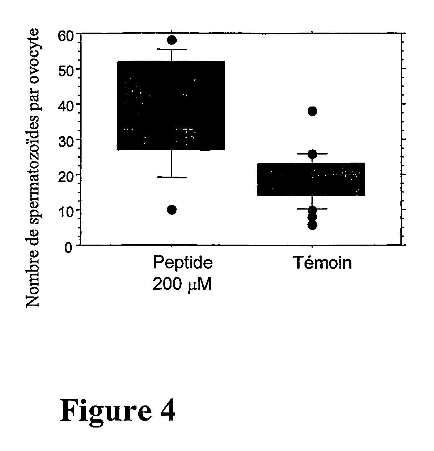

### Figure 4. Effets du Peptide FEEc sur la fécondance de l'ovocyte humain.

Comparaison des index de fécondation obtenus en absence et en présence du Peptide FEEc 100 µM dans le milieu d'incubation.

### Figure 5. Effets du Peptide QDEc sur la fusion gamétique chez la souris.

Les nombres entre parenthèse représentent le nombre d'ovocytes dans chaque groupe. Différence statistique avec les valeurs du groupe témoin : * (P<0,05) ; ** (P<0,04)

### Figure 6. Effet de QDE cyclique sur la fécondation in vitro d'ovocytes intacts de souris.

### REFERENCES

Accession Number : Souris Q60718 ; Homme Q99965; Cobaye Q60411 ; Lapin Q28660 ; Macaque Q28478 ; Bovin 077780 ; Rat Q63202 ; Porc CAC84225.
Aytoz A, Van den Abbeel E, Bonduelle M, Camus M, Joris H, Van Steirteghem A, Devroey P. Obstetric outcome of pregnancies after the transfer of cryopreserved and fresh embryos obtained by conventional in-vitro fertilization and intracytoplasmic sperm injection. Hum Reprod. 1999 Oct;14(10):2619-24.
Bavister BD.Early history of in vitro fertilization. Reproduction. 2002 Aug;124(2):181-96.
Bronson RA, Fusi FM, Calzi F, Doldi N, Ferrari A. Evidence that a functional fertilin-like ADAM plays a role in human sperm-oolemmal interactions. Mol Hum Reprod. 1999 May;5(5):433-40.
Evans JP. The molecular basis of sperm-oocyte membrane interactions during mammalian fertilization. Hum Reprod Update. 2002 Jul-Aug;8(4):297-311. Review.
Evans JP. Fertilin beta and other ADAMs as integrin ligands: insights into cell adhesion and fertilization. Bioessays. 2001 Jul;23(7):628-39. Review.
Evans JP, Schultz RM, and Kopf GS. Mouse sperm-egg plamsa membrane interactions: analysis of roles of egg integrins and the mouse sperm homologue of PH-30 (fertilin) β. J Cell Sci. 1995, 108, 3267-3278.
Gupta SK, Alves K, Palladino LO, Mark GE, Hollis GF. Molecular cloning of the human fertilin beta subunit. Biochem Biophys Res Commun. 1996 Jul 16;224(2):318-26.PMID: 8702389
Gupta S, Li H, Sampson NS. Characterization of fertilin beta-disintegrin binding specific in sperm-egg adhesion. Bioorg Med Chem 2000 Apr;8(4): 723-9
Kowalik A, Palermo GD, Barmat L, Veeck L, Rimarachin J, Rosenwaks Z. Comparison of clinical outcome after cryopreservation of embryos obtained from intracytoplasmic sperm injection and in-vitro fertilization. Hum Reprod. 1998 Oct;13(10):2848-51.
Li H, Sampson N. Structural analysis of fertilin(beta) cyclic peptide mimics that are ligands for alpha6betal integrin. J Pept Res 2002 Feb;59(2):45-54
Myles DG, Kimmel LH, Bolbel CP, White JM, and Primakoff P. Identification of a binding site in the disintegrin domain of fertilin required for sperm-egg fusion. Proc Natl Acad Sci USA 1994, 91, 4195-4198.
Mwethera PG, Makokha A, Chai D. Fertilin beta peptides inhibit sperm binding to zona-free egg in a homologous baboon in vitro fertilization system. Contraception 1999 Feb;59(2):131-5
Simon A, Holzer H, Hurwitz A, Revel A, Zentner BS, Lossos F, Laufer N. Comparison of cryopreservation outcome following intracytoplasmic sperm injection and conventional in vitro fertilization. J Assist Reprod Genet. 1998 Aug;15(7):431-7.
Swann K. Ca(2+) oscillations and sperm factors at fertilization in mammals. Hum Fertil (Camb). 1999;2(1):61-66.
Swann K, Parrington J, Jones KT. Potential role of a sperm-derived phospholipase C in triggering the egg-activating Ca2+ signal at fertilization. Reproduction. 2001 Dec;122(6):839-46. Review.
Tesarik J, Mendoza C, Greco E. Paternal effects acting during the first cell cycle of human preimplantation development after ICSI. Hum Reprod. 2002 Jan;17(1):184-9.
Vidaeus CM, von Kapp-Herr C, Golden WL, Eddy RL, Shows TB, Herr JC. uman fertilin beta: identification, characterization, and chromosomal mapping of an ADAM gene family member. Mol Reprod Dev. 1997 Mar;46(3):363-9.

## Revendications

1. Utilisation *in vitro* ou *ex vivo* d'un peptide cyclique comprenant le tripeptide formant un site de liaison de la fertiline béta à l'intégrine de l'ovocyte pour augmenter les capacités fusiogènes d'une gamète.

2. Utilisation selon la revendication 1, dans laquelle le tripeptide est X-(Q/D/E)-E, X étant un acide aminé.

3. Utilisation selon la revendication 1 ou 2, dans laquelle le tripeptide est FEE.

4. Utilisation selon l'une des revendications précédentes, dans laquelle ledit peptide présente la formule suivante : 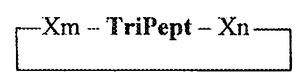 où X représente un acide aminé, m et n sont compris entre 0 et 14 et « TriPept » désigne ledit tripeptide.

5. Utilisation selon la revendication 4, dans laquelle m+n est inférieur à 10, de préférence inférieur ou égal à 5.

6. Utilisation selon la revendication 5, dans laquelle ledit peptide présente la formule suivante : 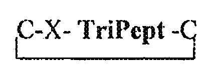 où X est un acide aminé.

7. Utilisation selon la revendication 6, dans laquelle X est un acide aminé peu encombrant et non chargé, de préférence choisi parmi A, S ou T.

8. Utilisation selon l'une des revendications précédentes, dans laquelle le peptide cyclique est sous forme de multimère.

9. Utilisation selon l'une des revendications précédentes, dans laquelle la gamète est un ovocyte.

10. Utilisation selon l'une des revendications 1 à 8, dans laquelle la gamète est un spermatozoïde.

11. Utilisation selon l'une des revendications précédentes destinée à améliorer la fécondation in vitro.

12. Utilisation selon l'une des revendications précédentes destinée à améliorer l'insémination artificielle, et/ou le transfert nucléaire chez des animaux non-humains.

13. Utilisation selon l'une des revendications 1 à 10 destinée à améliorer la fécondation in vitro et l'insémination artificielle chez les humains.

14. Peptide cyclique présentant la formule suivante : 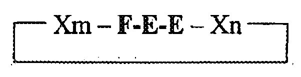 où X représente un acide aminé, m et n sont compris entre 0 et 14, un variant ou un dérivé de celui-ci.

15. Peptide cyclique selon la revendication 14, dans lequel m+n est inférieur à 10, de préférence inférieur ou égal à 5.

16. Peptide cyclique selon la revendication 15, ledit peptide présentant la formule suivante : 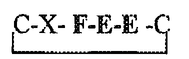 où X est un acide aminé, de préférence X étant choisi parmi A, S ou T.

17. Peptide cyclique selon la revendication 16, dans lequel X est S.

18. Multimère de peptides cycliques, lesdit peptides présentant la formule suivante : 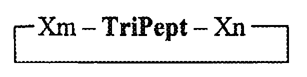 où X représente un acide aminé, m et n sont compris entre 0 et 14 et « TriPept » désigne le tripeptide X-(Q/D/E)-E formant un site de liaison de la fertiline béta à l'intégrine de l'ovocyte.

19. Multimère selon la revendication 18, dans lequel le tripeptide est FEE.

20. Multimère selon la revendication 18 ou 19, ledit peptide présentant la formule suivante : 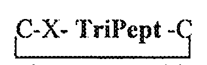 où X est un acide aminé, de préférence X étant choisi parmi A, S ou T.

21. Composition destinée à augmenter les capacités fusiogènes de gamètes comprenant un peptide selon l'une des revendications 14 à 17 ou un multimère selon l'une des revendications 18 à 20.

22. Utilisation d'un peptide selon l'une des revendications 14 à 17 ou un multimère selon l'une des revendications 18 à 20 pour la préparation d'un médicament destiné à traiter les problèmes de fécondité.

## Claims

1. Use *in vitro* or *ex vivo* of a cyclic peptide comprising the tripeptide forming a binding site of fertilin beta to oocyte integrin in order to increase the fusiogenic capacities of a gamete.

2. Use according to claim 1, wherein the tripeptide is X-(Q/D/E)-E, X being an amino acid.

3. Use according to either of claims 1 or 2, wherein the tripeptide is FEE.

4. Use according to any one of the previous claims, wherein said peptide has the following formula : 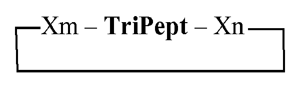 where X represents an amino acid, m and n are comprised between 0 and 14 and "TriPept" denotes said tripeptide.

5. Use according to claim 4, wherein m+n is less than 10, preferably less than or equal to 5.

6. Use according to claim 5, wherein said peptide has the following formula : 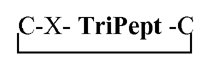 where X is an amino acid.

7. Use according to claim 6, wherein X is a small and uncharged amino acid, preferably selected in the group consisting of A, S or T.

8. Use according to any one of the previous claims, wherein the cyclic peptide is in the form of a multimer.

9. Use according to any one of the previous claims, wherein the gamete is an oocyte.

10. Use according to any one of claims 1-8, wherein the gamete is a spermatozoon.

11. Use according to any one of the previous claims, intended to improve in vitro fertilization.

12. Use according to any one of the previous claims, intended to improve artificial insemination, and/or nuclear transfer in non-human animals.

13. Use according to any one of claims 1-11 intended to improve in vitro fertilization and artificial insemination in humans.

14. Cyclic peptide having the following formula : 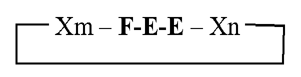 where X represents an amino acid, m and n are comprised between 0 and 14, a variant or a derivative thereof.

15. Cyclic peptide according to claim 14, wherein m+n is less than 10, preferably less than or equal to 5.

16. Cyclic peptide according to claim 15, said peptide having the following formula : 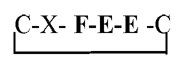 where X is an amino acid, X preferably being selected in the group consisting of A, S or T.

17. Cyclic peptide according to claim 16, wherein X is S.

18. Multimer of cyclic peptides, said peptides having the following formula : 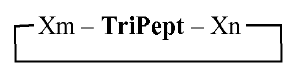 where X represents an amino acid, m and n are comprised between 0 and 14 and "TriPept" designates the tripeptide X-(Q/D/E)-E forming a binding site of fertilin beta to oocyte integrin.

19. Multimer according to claim 16, wherein the tripeptide is FEE.

20. Multimer according to either of claims 18 or 19, said peptide having the following formula : 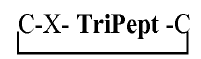 where X is an amino acid, X preferably being selected in the group consisting of A, S or T.

21. Composition intended for increasing fusiogenic capacities of gametes comprising a peptide according to any one of claims 14 to 17 or a multimer according to any one of claims 18 to 20.

22. Use of a peptide according to any one of claims 14 to 17 or a multimer according to any one of claims 18 to 20 for preparing a medicament intended to treat fertility problems.

## Patentansprüche

1. *In vitro* oder *ex vivo* Verwendung eines cyclischen Peptids, umfassend das Tripeptid, das eine Bindungsstelle des beta-Fertilins an das Integrin der Oozyte bildet, um die Fusionskapazitäten eines Gameten zu erhöhen.

2. Verwendung gemäß Anspruch 1, worin das Tripeptid X-(Q/D/E)-E ist, wobei X eine Aminosäure ist.

3. Verwendung gemäß Anspruch 1 oder 2, worin das Tripeptid FEE ist.

4. Verwendung gemäß einem der vorhergehenden Ansprüche, worin besagtes Peptid die folgende Formel aufweist: 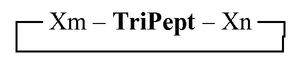 wo X für eine Aminosäure steht, m und n zwischen einschließlich 0 und 14 sind und "TriPept" besagtes Tripeptid bezeichnet.

5. Verwendung gemäß Anspruch 4, worin m + n kleiner als 10 ist, vorzugsweise kleiner oder gleich 5 ist.

6. Verwendung gemäß Anspruch 5, worin besagtes Peptid die folgende Formel aufweist: 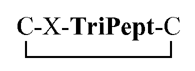 wo X eine Aminosäure ist.

7. Verwendung gemäß Anspruch 6, worin X eine wenig sperrige und ungeladene Aminosäure ist und vorzugsweise aus A, S oder T ausgewählt ist.

8. Verwendung gemäß einem der vorhergehenden Ansprüche, worin das cyclische Peptid in Form eines Multimers vorliegt.

9. Verwendung gemäß einem der vorhergehenden Ansprüche, worin der Gamet eine Oozyte ist.

10. Verwendung gemäß einem der Ansprüche 1 bis 8, worin der Gamet ein Spermatozoon ist.

11. Verwendung gemäß einem der vorhergehenden Ansprüche, die für eine Verbesserung der in vitro Befruchtung bestimmt ist.

12. Verwendung gemäß einem der vorhergehenden Ansprüche, die für eine Verbesserung der artifiziellen Insemination und/oder des Kemtransfers bei nicht-humanen Tieren bestimmt ist.

13. Verwendung gemäß einem der Ansprüche 1 bis 10, die für eine Verbesserung der in vitro Befruchtung und artifiziellen Insemination bei Menschen bestimmt ist.

14. Cyclisches Peptid, das die folgende Formel aufweist: 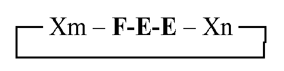 wo X für eine Aminosäure steht, m und n zwischen einschließlich 0 und 14 sind, sowie eine Variante oder ein Derivat davon.

15. Cyclisches Peptid gemäß Anspruch 14, in dem m + n kleiner als 10 ist, vorzugsweise kleiner oder gleich 5 ist.

16. Cyclisches Peptid gemäß Anspruch 15, wobei besagtes Peptid die folgende Formel aufweist: 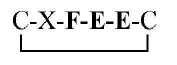 wo X eine Aminosäure ist, wobei X vorzugsweise aus A, S oder T ausgewählt ist.

17. Cyclisches Peptid gemäß Anspruch 16, in dem X S ist.

18. Multimere cyclischer Peptide, wobei besagte Peptide die folgende Formel aufweisen: 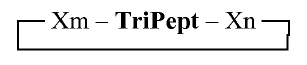 wo X für eine Aminosäure steht, m und n zwischen einschließlich 0 und 14 sind und "TriPept" das Tripeptid X-(Q/D/E)-E bezeichnet, das eine Bindungsstelle des beta-Fertilins an das Integrin der Oozyte bildet.

19. Multimer gemäß Anspruch 18, in dem das Tripeptid FEE ist.

20. Multimer gemäß Anspruch 18 oder 19, wobei besagtes Peptid die folgende Formel aufweist: 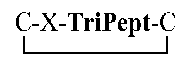 wo X eine Aminosäure ist, wobei X vorzugsweise aus A, S oder T ausgewählt ist.

21. Zusammensetzung, die für eine Erhöhung der Fusionskapazitäten von Gameten bestimmt ist und ein Peptid gemäß einem der Ansprüche 14 bis 17 oder ein Multimer gemäß einem der Ansprüche 18 bis 20 umfasst.

22. Verwendung eines Peptids gemäß einem der Ansprüche 14 bis 17 oder eines Multimers gemäß einem der Ansprüche 18 bis 20 für die Zubereitung eines Medikaments für die Behandlung von Problemen der Befruchtung.
